# FASCICULE DE BREVET EUROPEEN

(11) **EP 3 900 621 B1**
(45) Date de publication et mention de la délivrance du brevet: **31.08.2022**
(21) Numéro de dépôt: 21169927.7
(22) Date de dépôt: 22.04.2021
(51) Int. Cl.: A61B 5/026, A61B 5/024, A61B 5/145

(54) **DISPOSITIF DE SURVEILLANCE MEDICALE INDIVIDUELLE DU TYPE MONTRE CONNECTEE ET PROCEDE DE SURVEILLANCE MEDICALE INDIVIDUELLE D'UN UTILISATEUR CORRESPONDANT**
INDIVIDUELLES MEDIZINISCHES ÜBERWACHUNGSGERÄT VOM TYP VERBUNDENE UHR UND INDIVIDUELLES MEDIZINISCHES VERFAHREN ZUR ÜBERWACHUNG EINES ENTSPRECHENDEN NUTZERS
INDIVIDUAL MEDICAL MONITORING DEVICE SUCH AS A CONNECTED WATCH AND CORRESPONDING METHOD FOR INDIVIDUAL MEDICAL MONITORING OF A USER

(30) Priorité: 23.04.2020 FR 2004049
(43) Date de publication de la demande: 27.10.2021
(73) Titulaire: Kerrouche, Samira, 95140 Garges-lès-Gonesse (FR); Bouyahia, Hayame, 76520 Franqueville Saint Pierre (FR)
(72) Inventeur: Kerrouche, Samira, 95140 Garges-lès-Gonesse (FR); Bouyahia, Hayame, 76520 Franqueville Saint Pierre (FR)
(74) Mandataire: Ipside

(56) Documents cités:
- WO-A1-2018/114180
- US-A1- 2017 014 035
- US-A1- 2017 105 676
- US-A1- 2019 125 259
- US-A1- 2020 093 015

## Description

### DOMAINE TECHNIQUE

La présente invention se rapporte au domaine des dispositifs de surveillance médicale.

Plus particulièrement, l'invention concerne un dispositif de surveillance de personnes, notamment de personnes présentant un besoin de suivi médical, prenant la forme d'une montre destinée à être porté par un utilisateur au poignet et apte à délivrer une information caractéristique d'une anomalie en cas de danger pour l'utilisateur, notamment en cas de malaise ponctuel, de problème de santé plus grave ou de pathologie longue et traitées.

### ÉTAT DE L'ART

La surveillance de personnes souffrant de pathologies, telles que des pathologies chroniques, mais aussi de personnes fragiles ou de toutes personnes le souhaitant, est importante de sorte à pouvoir fournir une réponse adaptée et dans un temps imparti. Une telle surveillance se fait traditionnellement lors d'examens dans des centres spécialisés ou dans les hôpitaux.

Une telle surveillance peut en outre être faite directement par un patient, par exemple dans le cas de suivi post-traitement ou dans le cas d'un suivi quotidien pour des pathologies chroniques comme le diabète ou une maladie cardiaque.

Pour effectuer de tels suivis, il existe des dispositifs permettant de mesurer des paramètres physiologiques d'un patient tel que la glycémie ou le rythme cardiaque.

Des dispositifs de surveillance médicale individuelle de type montre connectée sont connus de US-2020/093015-A1, WO-2018/114180-A1, US-2017/105676-A1, US-2017/014035-A1 et US-2019/125259-A1.

Cependant, dans de trop nombreux cas, le suivi n'est pas assez régulièrement fait pour un patient qui peut oublier des prises des mesures ou ne pas se souvenir quand il a effectué une mesure ce qui peut avoir des conséquences plus ou moins graves.

En outre, un suivi non régulier d'un patient peut entraîner un décalage entre le traitement subi par le patient et celui qui lui serait nécessaire.

Par ailleurs, un tel suivi n'est pas précis du fait que, par exemple pour des maladies cardiaques, l'instant à laquelle une prise de mesure délivrant une information caractéristique d'une anomalie physiologique (une arythmie cardiaque, une perte de trop nombreux battements) peut s'avérer essentiel pour une prise en charge rapide et efficace d'une pathologie. Cependant, de trop nombreuses personnes (par exemple des personnes âgées) peuvent ne pas être en mesure de donner des détails suffisamment précis sur de tels phénomènes qui leur arriveraient. Ainsi, la réponse apportée à ce type d'incident peut ne pas être adéquat ce qui n'est pas satisfaisant.

De plus, un suivi en continu de personnes à risque peut également sauver la vie de patients et empêcher des frais médicaux (hospitalisation, traitement) évitables en permettant de prévenir d'éventuels problèmes de santés de ces personnes.

Il existe donc un besoin de fournir un dispositif de surveillance et de suivi médical qui permette de fournir un suivi en continu fiable pour que les patients puissent bénéficier d'une réponse adéquate à des symptômes.

Il existe également un besoin de fournir un tel dispositif qui soit simple d'utilisation et peu couteux à mettre en œuvre, de sorte que le plus grand nombre de patients puisse en bénéficier.

L'invention a notamment pour objectif de pallier les inconvénients de l'art antérieur.

### PRÉSENTATION DE L'INVENTION

L'invention répond à ce besoin en proposant un dispositif de surveillance médicale individuelle, de type montre connectée, apte à venir au contact d'un poignet d'un utilisateur, comprenant un corps principal muni d'un écran d'affichage, un bracelet raccordé au corps principal, et un module de mesure destiné à venir se positionner en vis-à-vis d'une artère radiale du poignet de l'utilisateur, lorsque la montre est portée par l'utilisateur, ledit module de mesure comprenant :
- un premier capteur apte à mesurer en continu le taux d'oxygène au niveau de l'artère radiale dudit utilisateur ;
- un deuxième capteur apte à mesurer le rythme cardiaque dudit utilisateur par mesure des vibrations du flux sanguin au niveau de ladite artère radiale ;
- un troisième capteur apte à mesurer les flux sanguins ascendant et descendant au niveau de ladite artère radiale,
ledit dispositif comprenant un module de traitement comportant :
- des moyens d'acquisition des données mesurées desdits premier capteur, deuxième capteur et troisième capteur ;
- des premiers moyens d'analyse du taux d'oxygène mesuré par ledit premier capteur, comparant ladite valeur du taux d'oxygène par rapport à un premier seuil prédéterminé ;
- des deuxièmes moyens d'analyse du nombre de vibrations du flux sanguin mesuré par ledit deuxième capteur, comparant ladite valeur du rythme cardiaque par rapport à un deuxième seuil prédéterminé ;
- des troisièmes moyens d'analyse des flux sanguins ascendant et descendant mesurés par ledit troisième capteur, comparant ladite valeur du flux sanguin ascendant par rapport à un troisième seuil prédéterminé et du flux sanguin descendant par rapport à un quatrième seuil prédéterminé ;
- des moyens de délivrance d'une information indiquant la détection ou la non détection d'une anomalie en fonction de ladite analyse desdits taux d'oxygène, rythme cardiaque et flux sanguins ascendant et descendant dudit utilisateur ;
- des moyens de stockage desdites données mesurées desdits premier capteur, deuxième capteur et troisième capteur.

Ainsi, l'invention propose une approche nouvelle et inventive permettant de résoudre au moins en partie certains des inconvénients de l'art antérieur.

Notamment, du fait que ce dispositif de surveillance médical individuelle peut être porté en continu, il permet de fournir un dispositif de surveillance et de suivi médical qui permette de fournir un suivi en continu fiable pour que les patients puissent bénéficier d'une réponse adéquate à des symptômes.

Par ailleurs, du fait que ces mesures sont effectuées au niveau de l'artère radiale de l'utilisateur, ce dispositif permet d'obtenir des données fiables et d'effectuer des analyses sur des données précises.

En outre, du fait de la pluralité de capteurs mis en œuvre ainsi que du module de traitement, les données peuvent également être recoupées ce qui amplifie cette fiabilité.

Un tel dispositif de surveillance médical individuelle s'avère en outre simple d'utilisation et peu couteux à mettre en œuvre.

Selon une caractéristique d'au moins un mode de réalisation de l'invention, lesdits moyens de stockage contiennent en outre au moins une information relative audit utilisateur appartenant au groupe comprenant :
- l'âge dudit utilisateur ;
- une donnée anatomique dudit utilisateur ;
- une donnée morphologique dudit utilisateur ;
- un traitement en cours pris par ledit utilisateur ;
- une pathologie dudit utilisateur ;
- les antécédents médicaux dudit utilisateur ;
- le groupe sanguin dudit utilisateur ;
- les ablations subies par l'utilisateur ;
- les opérations majeures subies par l'utilisateur ;
- le groupe sanguin de l'utilisateur ;
- les résultats de derniers examens (prise de sang, etc.).

Cela permet ainsi de fournir des données encore plus précises.

Dans ce cas de figure, selon une caractéristique d'au moins un mode de réalisation de l'invention :
- le premier seuil prédéterminé est défini en fonction de ladite moins une information relative audit utilisateur ; et/ou
- le deuxième seuil prédéterminé est défini en fonction de ladite moins une information relative audit utilisateur ; et/ou
- le troisième seuil prédéterminé est défini en fonction de ladite moins une information relative audit utilisateur ; et/ou
- le quatrième seuil prédéterminé est défini en fonction de ladite moins une information relative audit utilisateur.

Selon une caractéristique d'au moins un mode de réalisation de l'invention, le dispositif comprend en outre des moyens d'alerte. En outre, si une information indiquant la détection d'une anomalie est délivrée par lesdits moyens de délivrance, lesdits moyens d'alerte génèrent un message d'alerte qui est transmis par le biais de moyens d'émission à au moins un contact prédéterminé, ledit contact prédéterminé étant stocké dans lesdits moyens de stockage.

Selon une caractéristique d'au moins un mode de réalisation de l'invention, lesdits moyens d'émission comprennent un port configuré pour recevoir une carte nano SIM.

Ainsi, cela permet de mettre en œuvre des moyens de communication simples, et accessibles à tous. En outre, cela permet d'éviter d'éventuels problèmes de compatibilité de moyens de communication.

Selon une caractéristique d'au moins un mode de réalisation de l'invention, lesdits moyens d'émission comprennent en outre des moyens d'appel d'urgence dudit au moins un contact prédéterminé.

Ainsi, cela permet de pouvoir fournir une réponse rapide en cas d'urgence.

Selon une caractéristique d'au moins un mode de réalisation de l'invention, le dispositif comprend en outre des moyens d'identification uniques.

De ce fait, cela permet de sécuriser les données contenues par ce dispositif de sorte que celles-ci ne soient pas accessibles à tous. En outre, cela permet de s'assurer que les données correspondent bien à une personne donnée.

Selon une caractéristique d'au moins un mode de réalisation de l'invention, le dispositif comprend en outre des moyens de détection d'une mise en place dudit dispositif sur ledit poignet dudit utilisateur.

De ce fait, cela permet d'éviter de fausses mesures et donc de fausses alertes en s'assurant que le dispositif est bien en place sur le poignet de l'utilisateur.

Selon une caractéristique d'au moins un mode de réalisation de l'invention, le dispositif comprend en outre un capteur appartient au groupe comprenant :
- un capteur de géolocalisation ;
- un capteur de glycémie ;
- un capteur de température.

Selon une caractéristique d'au moins un mode de réalisation de l'invention, le dispositif comprend en outre une alimentation électrique.

Cela permet ainsi de mettre en œuvre un dispositif qui est autonome d'un point de vue énergétique, moyennant des recharges éventuelles.

L'invention concerne également un procédé de surveillance médicale individuelle, ledit procédé étant apte à délivrer une information indiquant la détection ou la non détection d'une anomalie, mettant en œuvre un dispositif de surveillance médicale individuelle, de type montre connectée apte à venir au contact d'un poignet d'un utilisateur selon l'un des modes de réalisation précités, le procédé comprenant les étapes suivantes, mises en œuvre par ledit dispositif de surveillance médicale individuelle, lorsque ledit module de mesure est positionné en vis-à-vis d'une artère radiale dudit poignet dudit utilisateur :
- mesure du taux d'oxygène dudit utilisateur au niveau de l'artère radiale dudit utilisateur, à l'aide dudit premier capteur ;
- mesure du rythme cardiaque dudit utilisateur par mesure des vibrations du flux sanguin au niveau de ladite artère radiale, à l'aide dudit deuxième capteur ;
- mesure des flux sanguins ascendant et descendant au niveau de ladite artère radiale, à l'aide dudit troisième capteur ;
- analyse desdits taux d'oxygène, rythme cardiaque et flux sanguins ascendant et descendant dudit utilisateur, de sorte à transmettre des instructions de délivrance d'une information indiquant la détection ou la non détection d'une anomalie ;
- délivrance de ladite information indiquant la détection ou la non détection d'une anomalie ;
- stockage desdits taux d'oxygène, rythme cardiaque et flux sanguins ascendant et descendant mesurés dudit utilisateur ;
ladite étape d'analyse comprenant les étapes successives suivantes :
- comparaison du taux d'oxygène mesuré par rapport à un premier seuil prédéterminé ;
- comparaison du rythme cardiaque mesuré par rapport à un deuxième seuil prédéterminé ;
- comparaison de la valeur du flux sanguin ascendant par rapport à un troisième seuil prédéterminé et de la valeur du flux sanguin descendant par rapport à un quatrième seuil prédéterminé ; et
- instruction de délivrance de ladite information indiquant la détection d'une anomalie si le taux d'oxygène, et/ou le rythme cardiaque et/ou les flux sanguins ascendant et/ou descendant dudit utilisateur dépassent respectivement les premier, deuxième, troisième et quatrième seuils.

Selon une caractéristique d'au moins un mode de réalisation du procédé, si une information indiquant la détection d'une anomalie est délivrée par lesdits moyens de délivrance, le procédé comprend les étapes successives suivantes :
- génération d'un message d'alerte par lesdits moyens d'alerte ;
- envoi dudit message d'alerte généré audit au moins un contact prédéterminé, par le biais desdits moyens d'émission.

Selon une caractéristique d'au moins un mode de réalisation du procédé, celui-ci comprend une étape préalable d'authentification.

L'invention concerne en outre un produit programme d'ordinateur téléchargeable depuis un réseau de communication et/ou stocké sur un support lisible par microprocesseur et/ou exécutable par un microprocesseur, caractérisé en ce qu'il comprend des instructions de code de programme pour l'exécution d'un procédé de surveillance médicale individuelle selon l'un des modes de réalisation précités, lorsqu'il est exécuté sur un ordinateur ou un terminal mobile.

L'invention concerne encore un médium de stockage lisible par un terminal et non transitoire, stockant un programme d'ordinateur comprenant un jeu d'instructions exécutables par un ordinateur ou un processeur pour mettre en œuvre le procédé selon l'un des modes de réalisation précités.

### BRÈVE DESCRIPTION DES FIGURES

D'autres buts, caractéristiques et avantages de l'invention apparaîtront plus clairement à la lecture de la description suivante, donnée à titre de simple exemple illustratif, et non limitatif, en relation avec les figures, parmi lesquelles :
- Figure 1 : est un schéma illustrant en vue de côté un dispositif de surveillance selon un mode de réalisation ;
- Figure 2 : est un schéma illustrant un module de mesure et un module de traitement selon un mode de réalisation de l'invention ;
- Figure 3 : est un schéma illustrant un procédé de surveillance d'un utilisateur selon un mode de réalisation de l'invention ; et
- Figure 4 : est un schéma illustrant en détail l'étape d'analyse du procédé de suivi selon le mode de réalisation de la figure 3.

### DESCRIPTION DÉTAILLÉE DE MODES DE RÉALISATION

Le principe général de l'invention repose sur la mise en œuvre d'un dispositif de surveillance médicale individuelle, de type montre connectée, apte à venir au contact d'un poignet d'un utilisateur, comprenant un corps principal muni d'un écran d'affichage, un bracelet raccordé au corps principal, et un module de mesure destiné à venir se positionner en vis-à-vis d'une artère radiale du poignet de l'utilisateur, lorsque la montre est portée par l'utilisateur, et pouvant indiquer la détection ou la non détection d'une anomalie en fonction de plusieurs critères qui sont mesurés au niveau de cette artère radiale.

Une telle montre permet d'effectuer une analyse cohérente des données vitales grâce à une combinaison de capteurs, et ainsi déterminer l'état d'une personne. Cette détermination peut par exemple permettre d'effectuer des diagnostics en direct par des services de secours, d'effectuer des téléconsultations, d'effectuer de la surveillance de personnes âgées.

Un tel dispositif peut être utilisé pour renforcer la sécurité et la surveillance de personnes, plus particulièrement de personnes vulnérables.

Un tel dispositif de surveillance individuelle peut, par exemple, être utilisé pour la surveillance de patients présentant des pathologies chroniques ou devant faire l'objet d'un suivi particulier (par exemple un suivi post hospitalisation). Un tel dispositif peut également s'avérer utile pour le suivi médical de personnes âgées.

Ce type de dispositif s'avère simple de conception et d'utilisation.

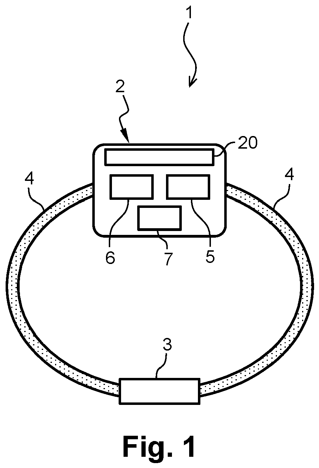

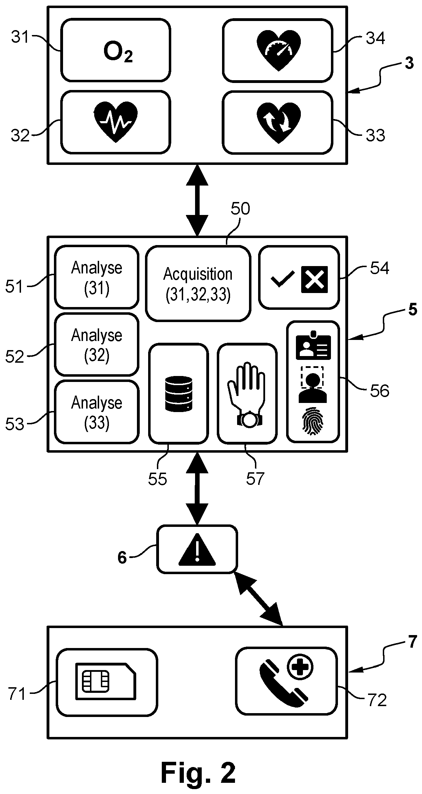

On présente maintenant, en relation avec les figures 1 et 2, un premier mode de réalisation du dispositif de surveillance selon l'invention.

Comme illustré sur ces figures, le dispositif 1 de surveillance médicale individuelle est de type montre connectée. Il est apte à venir au contact d'un poignet d'un utilisateur, de sorte que l'une des portions, plus particulièrement la portion du dispositif où est logé un module de mesure 3, puisse venir se positionner en vis-à-vis d'une artère radiale du poignet de l'utilisateur, lorsque la montre est portée par cet utilisateur. Cette montre comprend en outre un corps principal 2 muni d'un écran d'affichage 20 et un bracelet 4 raccordé au corps principal 2.

Un tel corps principal 2 est, dans ce mode de réalisation, réalisé par moulage de plastique injecté de sorte à fournir un matériau étanche et résistant aux différentes conditions dans lesquelles il pourrait être amené à évoluer, tel que l'eau d'une douche ou d'une baignoire (afin que l'utilisateur puisse conserver sa montre connectée en permanence.

On pourrait également prévoir un corps principal réalisé en un autre matériau qui permette de fournir les mêmes conditions de résistance et d'étanchéité.

Le bracelet 4 peut également être réalisé par moulage de plastique injecté ou en silicone.

Ce bracelet peut, par exemple, présenter une fermeture positionnée sur un côté, de sorte à faciliter sa mise et son retrait.

Toutefois, le bracelet pourrait également être un bracelet à fermoir ou un bracelet à verrouillage pour des questions de sécurité.

Il est à noter que le dispositif présenté dans ce mode de réalisation comprend une alimentation électrique. Une telle alimentation se présente ici sous la forme d'une batterie, ménagée au niveau des moyens de traitement 5, qui permet ainsi à ce dispositif d'être autonome.

Cette batterie peut, selon un mode de réalisation, être une pile rechargeable ou non. Une telle batterie pourrait, en outre, être une batterie LiPO permettant une charge rapide. Cette batterie peut être reliée à un port USB permettant de la recharger. Le rechargement pourrait également se faire par induction de sorte à éviter une entrée potentielle d'eau ou de poussière.

Le rechargement d'une telle batterie pourrait également être effectué de manière solaire, de sorte à fournir une solution écologique.

Comme décrit précédemment, le dispositif de surveillance comprend un module de mesure 3. Dans ce mode de réalisation, ce module de mesure 3 comprend :
- un premier capteur 31 apte à mesurer en continu le taux d'oxygène de l'utilisateur au niveau de l'artère radiale ;
- un deuxième capteur 32 apte à mesurer le rythme cardiaque de l'utilisateur par mesure des vibrations au niveau de l'artère radiale ;
- un troisième capteur 33 apte à mesurer les flux sanguins ascendant et descendant au niveau de l'artère radiale.

Dans ce mode de réalisation, et de sorte à avoir un suivi permanent de l'utilisateur, ces mesures sont effectuées en continu.

Bien évidemment, le cas échéant, on pourrait prévoir que l'une ou l'autre de ces mesures soit effectuée de manière périodique de sorte, par exemple, à économiser la batterie de la montre connectée.

Il est à noter que le premier capteur 31 mesure le taux d'oxygène de l'utilisateur par le biais d'un élément équipée d'une lumière, le taux d'oxygène étant ensuite évalué par un algorithme mis en œuvre à l'endroit du module de traitement (présenté ci-après).

Ce capteur d'oxygène peut par exemple, mettre en œuvre des méthodes de pléthysmographie miniature.

Dans ce mode de réalisation, le module de mesure comprend en outre un capteur de tension 34 permettant de mesure la tension de l'utilisateur au niveau du poignet. Il est à noter que, selon un mode de réalisation de l'invention, un tel dispositif pourrait également être connecté à un tensiomètre de sorte que les mesures de tension soient prises et conservées sur une période de temps prédéfinie. Cela permettrait ainsi d'avoir un état d'un patient sur cette période de temps prédéfinie. Une telle variante pourrait être un complément au capteur de tension, en cas de besoin d'une vérification des données.

Il est à noter également qu'un tel tensiomètre pourrait être apte à communiquer avec ce dispositif de surveillance, par des moyens de communication sans fil classiques.

Selon un mode de réalisation non représenté, ce module de mesure pourrait également comprendre un capteur permettant de détecter une chute de l'utilisateur, par exemple un altimètre apte à détecter un changement brutal d'altitude (ici, d'altitude du poignet).

Selon un autre mode de réalisation non représenté, le module de mesure pourrait également comprendre un capteur permettant de mesurer la glycémie de l'utilisateur.

Selon un autre mode de réalisation non représenté, le module de mesure pourrait également comprendre un capteur permettant de mesurer la température de l'utilisateur.

Selon un autre mode de réalisation non représenté, le dispositif pourrait également comprendre un capteur de géolocalisation. Un tel capteur pourrait, par exemple, s'avérer utile dans le cadre de la prévention ou de l'action pour des personnes qui seraient atteintes de la maladie d'Alzheimer, des personnes âgées, ou des personnes atteintes de pathologie psychiatrique.

De sorte à privilégier des capteurs essentiels surveillant les fonctions vitales, il est à noter qu'un ou plusieurs des capteurs additionnels peuvent, selon un mode de réalisation, être activés ou désactiver par action de l'utilisateur, par exemple par action tactile sur l'écran d'affichage 20 ou par une combinaison de boutons.

Dans ce mode de réalisation, le module de mesure 3 est relié, par le biais d'un câble, qui est ici un câble plat, à un module de traitement 5 qui a pour but d'analyser les paramètres mesurés et de délivrer une information indiquant la détection ou la non détection d'une anomalie en fonction de ladite analyse.

Ce module de traitement 5 comporte ici :
- des moyens 50 d'acquisition des données mesurées des premier capteur 31, deuxième capteur 32 et troisième capteur 33 ;
- des premiers moyens 51 d'analyse du taux d'oxygène mesuré par le premier capteur 31, positionné au niveau de l'artère radiale comparant la valeur du taux d'oxygène par rapport à un premier seuil prédéterminé ;
- des deuxièmes moyens 52 d'analyse du nombre de vibrations mesuré par le deuxième capteur 32, comparant la valeur du rythme cardiaque par rapport à un deuxième seuil prédéterminé ;
- des troisièmes moyens 53 d'analyse des flux sanguins ascendant et descendant mesurés par le troisième capteur 33, comparant la valeur du flux sanguin ascendant par rapport à un troisième seuil prédéterminé et du flux sanguin descendant par rapport à un quatrième seuil prédéterminé ; et
- des moyens de délivrance 54 d'une information indiquant la détection ou la non-détection d'une anomalie en fonction de l'analyse des taux d'oxygène, rythme cardiaque et flux sanguins ascendant et descendant de l'utilisateur.

Selon un mode de réalisation de l'invention, les moyens de délivrance 54 d'une information indiquant la détection ou la non détection d'une anomalie en fonction de l'analyse des taux d'oxygène, rythme cardiaque et flux sanguins ascendant et descendant de l'utilisateur peuvent délivrer une information indiquant la détection d'une anomalie si au moins l'un des paramètres mesurés est caractéristique d'une anomalie.

Par exemple, en cas de baisse brutale du taux d'oxygène, visible par la mesure du taux d'oxygène puis la comparaison par rapport à un seuil prédéterminé de taux d'oxygène attendu, les moyens de délivrance peuvent délivrer une information indiquant la détection d'une anomalie.

En outre, si la comparaison de la valeur du flux sanguin ascendant par rapport à un troisième seuil prédéterminé et/ou du flux sanguin descendant par rapport à un quatrième seuil prédéterminé révèle une anomalie, cela peut être révélateur d'une pathologie cardiaque (arythmie, tachycardie, bradycardie, ou malaise cardiaque), les moyens de délivrance peuvent donc également délivrer une information indiquant la détection d'une anomalie.

Selon un autre mode de réalisation, et de sorte à pouvoir réduire la possibilité d'une fausse alerte, les moyens de délivrance peuvent délivrer une information indiquant la détection d'une anomalie si au moins deux des paramètres mesurés sont caractéristiques d'une anomalie, c'est-à-dire si au moins deux des paramètres mesurés sont en dehors du seuil prédéterminé respectif.

Il est à noter que ce module de traitement 5 comprend en outre des moyens 55 de stockage des données mesurées des premier capteur 31, deuxième capteur 32 et troisième capteur 33.

Le fait de stocker les données mesurées des premier capteur 31, deuxième capteur 32 et troisième capteur 33 permet de conserver un historique des information vitales de l'utilisateur sur une période plus ou moins longue selon les modes de réalisation. De cette manière, une personne qui consulte l'historique, tel qu'un médecin ou une infirmière, peut avoir une vision complète de l'historique médical de l'utilisateur de la montre.

Selon un mode de réalisation, le dispositif comprend en outre des moyens d'analyse des données mesurées et stockées sur une période prédéfinie, par exemple pour détecter d'éventuelles anomalies, telle qu'une arythmie cardiaque.

Cela peut également être utile dans le cadre de la détection de chutes, une analyse des données entre deux mesures successives pouvant permettre de détecter un changement brutal d'altitude du poignet, pouvant être synonyme d'une chute. Cela peut également être utile dans le cadre de la surveillance du diabète, une analyse des données entre plusieurs mesures successives pouvant permettre de détecter un changement brutal du taux de sucre dans le sang de l'utilisateur, pouvant être synonyme d'une hypoglycémie ou d'une hyperglycémie.

Il est à noter que, selon le mode de réalisation présenté ici, les moyens 55 de stockage contiennent en outre au moins une information relative à l'utilisateur appartenant au groupe comprenant :
- l'âge de l'utilisateur ;
- une donnée anatomique de l'utilisateur ;
- une donnée morphologique de l'utilisateur ;
- un traitement en cours pris par l'utilisateur ;
- une pathologie de l'utilisateur ;
- les antécédents médicaux de l'utilisateur ;
- les ablations subies par l'utilisateur ;
- les opérations majeures subies par l'utilisateur ;
- le groupe sanguin de l'utilisateur ;
- les résultats de derniers examens (prise de sang, etc.).

Dans ce cas, et de manière à ce que la réponse apportée à une éventuelle anomalie soit la plus adaptée possible, il est préférable que :
- le premier seuil prédéterminé soit défini en fonction de ladite moins une information relative audit utilisateur ;
- le deuxième seuil prédéterminé soit défini en fonction de ladite moins une information relative audit utilisateur ;
- le troisième seuil prédéterminé soit défini en fonction de ladite moins une information relative audit utilisateur ; et/ou
- le quatrième seuil prédéterminé soit défini en fonction de ladite moins une information relative audit utilisateur.

Par exemple, un seuil de rythme cardiaque ne sera pas identique que la personne soit sportive ou non, selon son âge ou sa morphologie.

De même, un traitement en cours peut changer les critères d'alerte. Par exemple, un traitement contre la tension peut faire varier le seuil auquel une tension est mesurée s'avère être une anomalie.

Il est à noter que, selon un mode de réalisation, cette ou ces informations relatives à l'utilisateur peuvent être consultées par affichage sur l'écran d'affichage 20 du corps principal 2.

Cela peut par exemple être accessible par une combinaison de touches ou par appui sur cet écran d'affichage.

De cette manière, cela peut permettre d'afficher des traitements médicaux en cours.

Selon un mode de réalisation, un ou plusieurs des informations relatives à l'utilisateur peuvent être automatiquement affichées en cas de délivrance d'une information indiquant la détection d'une anomalie. Une telle caractéristique peut par exemple s'avérer utile dans le cas d'intervention des secours qui peuvent avoir besoin d'accéder à certaines données d'un patient pour pouvoir intervenir rapidement.

Comme illustré sur la figure 2, le dispositif selon ce mode de réalisation comprend en outre des moyens d'alerte 6. De ce fait, si une information indiquant la détection d'une anomalie est délivrée par les moyens de délivrance 54, ces moyens d'alerte 6 génèrent un message d'alerte qui est transmis par le biais de moyens d'émission 7 à au moins un contact prédéterminé.

Ici, les moyens d'émission 7 comprennent un port 71 configuré pour recevoir une carte SIM, de préférence une carte nano SIM.

Ces moyens d'émission peuvent être accompagnés de moyens de transmission mis en œuvre sous la forme d'une antenne fonctionnant par technologie sans fil tel que Bluetooth, Wifi, 3G, 4G, ou 5G.

De cette manière, le message d'alerte peut, par exemple, être émis sous la forme d'un SMS ou d'un message vocal envoyé sur le téléphone d'une personne.

Ce message d'alerte peut également être émis sous la forme d'une notification sur un terminal mobile du contact prédéterminé.

Ce message d'alerte peut encore être accompagné d'une alerte envoyée à des services de secours de sorte à gagner en efficacité pour l'intervention.

Selon un mode de réalisation, le message d'alerte peut contenir des informations importantes sur l'alerte générée, par exemple la mesure qui a généré cette alerte, des informations relatives à l'utilisateur (tel qu'un éventuel traitement en cours ou une pathologie).

Selon un mode de réalisation, le message d'alerte peut également être accompagné d'un pré-diagnostic de sorte à aiguiller plus facilement une personne vers le bon service de secours.

Dans le mode de réalisation présenté, le ou les contacts prédéterminés sont stockés dans les moyens 55 de stockage.

Selon un mode de réalisation de l'invention, ce contact prédéterminé peut également être automatiquement affiché sur l'écran d'affichage 20 en cas de délivrance d'une information indiquant la détection d'une anomalie.

On peut prévoir un mode de réalisation de l'invention dans lequel les données mesurées sont accessibles par l'utilisateur qui, par exemple par action sur l'écran d'affichage ou par combinaison de boutons, envoie des données à un praticien médical par le biais des moyens d'émission 7 de sorte à pouvoir effectuer une consultation à distance.

On peut également prévoir un mode de réalisation dans lequel les moyens d'émission sont accompagnés de moyens de réception (non représentés ici) de sorte qu'un tiers, par exemple un praticien médical, puisse saisir des données à un format reconnu par le dispositif. De cette manière, un praticien médical pourrait, par exemple, effectuer une prescription médicale à distance, le traitement entrant alors directement comme information relative à l'utilisateur.

On peut également prévoir que le dispositif de surveillance comprenne des moyens d'émission d'une notification sur l'écran digital de sorte à rappeler à l'utilisateur un traitement à prendre ou une consultation planifiée.

De manière avantageuse, le dispositif de surveillance selon le mode de réalisation présenté comprend en outre des moyens 56 d'identification uniques.

De cette manière, cela permet de sécuriser le dispositif en limitant l'accès aux données et à son utilisation à une ou plusieurs personnes autorisées.

Ici, l'identification de l'utilisateur se fait par la saisie du numéro de sécurité sociale de l'utilisateur.

Toutefois, on pourrait prévoir un mode de réalisation dans lequel le moyen d'identification pourrait être un autre moyen, tel qu'une empreinte digitale ou une reconnaissance faciale.

On pourrait également prévoir un mode de réalisation dans lequel ces moyens d'identification uniques comprennent un mot de passe.

En outre, le dispositif selon le mode de réalisation présenté comprend des moyens 57 de détection d'une mise en place de ce dispositif sur le poignet de l'utilisateur. De cette manière, cela permet d'éviter des fausses alertes dans le cas d'une mesure suspecte alors que le dispositif ne serait pas placé sur le poignet de l'utilisateur. Dans le mode de réalisation illustré, les moyens d'émission comprennent en outre des moyens 72 d'appel d'urgence du contact prédéterminé.

Ces moyens d'appel d'urgence peuvent être mis en œuvre sous la forme de deux boutons placés de part et d'autre du corps principal, l'utilisateur devant appuyer de manière simultanée sur les deux boutons pour effectuer cet appel d'urgence.

De tels moyens d'appel d'urgence peuvent également être mis en œuvre sous la forme d'un unique bouton, par exemple un bouton placé à un endroit peu accessible du dispositif de sorte qu'il ne puisse pas être utilisé de manière fortuite.

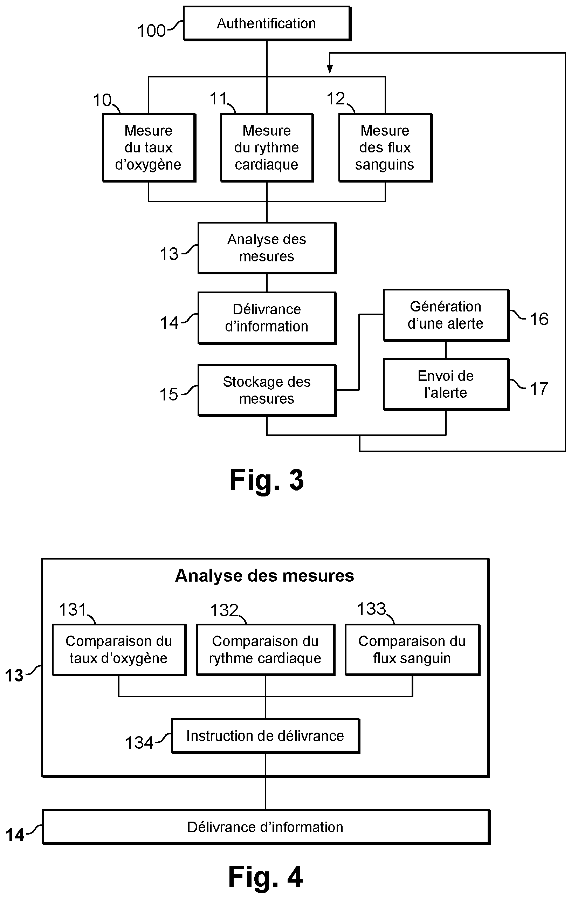

On présente maintenant, en relation avec les figures 3 et 4, un procédé de surveillance médicale individuelle, ce procédé étant apte à délivrer une information indiquant la détection ou la non détection d'une anomalie.

Selon l'invention, ce procédé de surveillance médicale individuelle met en œuvre un dispositif 1 de surveillance médicale individuelle, de type montre connectée apte à venir au contact d'un poignet d'un utilisateur selon l'un des modes de réalisation précités.

Comme illustré, le procédé comprend les étapes suivantes, mises en œuvre par la montre connectée, lorsque le module 3 de mesure est positionné en vis-à-vis d'une artère radiale du poignet de l'utilisateur :
- mesure 10 du taux d'oxygène de l'utilisateur, à l'aide du premier capteur 31 positionné au niveau de l'artère radial ;
- mesure 11 du rythme cardiaque de l'utilisateur par mesure des vibrations du flux sanguin au niveau de l'artère radiale, à l'aide du deuxième capteur 32 ;
- mesure 12 des flux sanguins ascendant et descendant au niveau de l'artère radiale, à l'aide du troisième capteur 33 ;
- analyse 13 des taux d'oxygène, rythme cardiaque et flux sanguins ascendant et descendant de l'utilisateur, de sorte à transmettre des instructions de délivrance d'une information indiquant la détection ou la non détection d'une anomalie ;
- délivrance 14 de l'information indiquant la détection ou la non détection d'une anomalie ;
- stockage 15 des taux d'oxygène, rythme cardiaque et flux sanguins ascendant et descendant mesurés de l'utilisateur.

Selon l'invention, l'étape d'analyse 13 comprend les étapes successives suivantes :
- comparaison 131 du taux d'oxygène mesuré par rapport à un premier seuil prédéterminé ;
- comparaison 132 du rythme cardiaque mesuré par rapport à un deuxième seuil prédéterminé ;
- comparaison 133 de la valeur du flux sanguin ascendant par rapport à un troisième seuil prédéterminé et de la valeur du flux sanguin descendant par rapport à un quatrième seuil prédéterminé ; et
- instruction de délivrance 134 de l'information indiquant la détection d'une anomalie si le taux d'oxygène, et/ou le rythme cardiaque et/ou les flux sanguins ascendant et /ou descendant de l'utilisateur dépassent respectivement les premier, et/ou deuxième, et/ou troisième et/ou quatrième seuils.

Selon un mode de réalisation de l'invention, l'étape d'instructions de délivrance 14 d'une information indiquant la détection ou la non détection d'une anomalie en fonction de l'analyse des taux d'oxygène, et/ou rythme cardiaque et/ou flux sanguins ascendant et/ou descendant de l'utilisateur peut délivrer une information indiquant la détection d'une anomalie si au moins l'un des paramètres mesurés est caractéristique d'une anomalie.

Par exemple, en cas de baisse brutale du taux d'oxygène, visible par la mesure du taux d'oxygène puis la comparaison par rapport à un seuil prédéterminé de taux d'oxygène attendu, les moyens de délivrance peuvent délivrer une information indiquant la détection d'une anomalie.

En outre, si la comparaison de la valeur du flux sanguin ascendant par rapport à un troisième seuil prédéterminé et/ou du flux sanguin descendant par rapport à un quatrième seuil prédéterminé révèle une anomalie, cela peut être révélateur d'une pathologie cardiaque (arythmie, tachycardie, bradycardie, ou malaise cardiaque), les moyens de délivrance peuvent donc également délivrer cette information indiquant la détection d'une anomalie.

Selon un autre mode de réalisation, et de sorte à pouvoir réduire la possibilité d'une fausse alerte, l'étape de délivrance pouvant délivrer une information indiquant la détection d'une anomalie peut être effectuée si au moins deux des paramètres mesurés sont caractéristiques d'une anomalie, c'est-à-dire si au moins deux des paramètres mesurés sont en dehors du seuil prédéterminé respectif.

Dans le mode de réalisation du procédé illustré en figure 3, si une information indiquant la détection d'une anomalie est délivrée par les moyens de délivrance 54, le procédé comprend les étapes successives suivantes :
- génération 16 d'un message d'alerte par les moyens d'alerte 6 ;
- envoi 17 du message d'alerte généré au contact prédéterminé, par le biais des moyens d'émission.

En outre, dans le mode de réalisation illustré, le procédé comprend une étape 100 préalable d'authentification. De cette manière, cela permet de sécuriser le dispositif en limitant l'accès aux données et à son utilisation à une ou plusieurs personnes autorisées.

L'invention concerne également un produit programme d'ordinateur téléchargeable depuis un réseau de communication et/ou stocké sur un support lisible par microprocesseur et/ou exécutable par un microprocesseur, comprenant des instructions de code de programme pour l'exécution d'un procédé de surveillance médicale individuelle selon l'un des modes de réalisation précités, lorsqu'il est exécuté sur un ordinateur.

L'invention concerne également un médium de stockage lisible par ordinateur et non transitoire, stockant un programme d'ordinateur comprenant un jeu d'instructions exécutables par un ordinateur ou un processeur pour mettre en œuvre le procédé de surveillance médicale individuelle selon l'un des modes de réalisation précités.

Plus particulièrement, le médium de stockage peut être inclus dans le corps principal, par exemple dans la mémoire 55 intégrée à l'endroit du module de traitement. Il pourrait également être inclus à l'endroit du module de mesure. Selon un mode de réalisation, on pourrait prévoir que le dispositif et le procédé soient accompagnés de la création d'une carte associée pour un lecteur (par exemple une carte SD ou une carte à puce qui contiendrait également l'ensemble des informations stockées dans la mémoire intégrée. Une telle copie de donnée peut se faire par le biais d'une synchronisation en ligne ou par le biais des moyens d'émission.

On pourrait en outre prévoir qu'une telle carte associée puisse être utilisable en lien avec un logiciel permettant de synchroniser l'ensemble de sorte à générer un dossier médical complet pour un patient (incluant par exemple les informations invariables du patient, le détail des antécédents médicaux du patient, un enregistrement des ordonnances prescrites, des résultats de radiologie, des résultats de prises de sang permettant une lecture immédiate d'une situation relative au patient).

Par exemple, on pourrait prévoir que le logiciel permette d'avoir en automatique entre le laboratoire et un ou plusieurs médecins la transmission des informations par catégorie et une synthèse globale des données sanguines par mois voire par année, sans avoir de relecture papier.

On pourrait également prévoir que la mise à jour des examens puisse être directement transmise par le médecin à l'utilisateur, par exemple après validation par l'utilisateur de l'accès à sa montre connectée pour enregistrer ces informations. On pourrait encore prévoir des moyens de synchronisation entre le dispositif de surveillance, la carte associée, et le dossier médical complet du patient stocké dans une base de données.

On pourrait également prévoir qu'un tel dispositif comprenne un échéancier de maintenance, de sorte à ce que les fonctions offertes restent optimales et que le suivi médical soit fiable pour un utilisateur donné.

## Revendications

1. Dispositif (1) de surveillance médicale individuelle, de type montre connectée, apte à venir au contact d'un poignet d'un utilisateur, comprenant un corps principal (2) muni d'un écran d'affichage (20), un bracelet (4) raccordé au corps principal (2), et un module de mesure (3) destiné à venir se positionner en vis-à-vis d'une artère radiale du poignet de l'utilisateur, lorsque la montre est portée par l'utilisateur, ledit module de mesure (3) comprenant :
- un premier capteur (31) apte à mesurer en continu le taux d'oxygène au niveau de l'artère radiale dudit utilisateur ;
- un deuxième capteur (32) apte à mesurer le rythme cardiaque dudit utilisateur par mesure des vibrations du flux sanguin au niveau de ladite artère radiale ;
- un troisième capteur (33) apte à mesurer les flux sanguins ascendant et descendant au niveau de ladite artère radiale ;
ledit dispositif (1) comprenant un module de traitement (5) comportant :
- des moyens (50) d'acquisition des données mesurées desdits premier capteur (31), deuxième capteur (32) et troisième capteur (33) ;
- des premiers moyens (51) d'analyse du taux d'oxygène mesuré par ledit premier capteur (31), aptes à comparer ladite valeur du taux d'oxygène par rapport à un premier seuil prédéterminé ;
- des deuxièmes moyens (52) d'analyse du nombre de vibrations du flux sanguin mesuré par ledit deuxième capteur (32), aptes à comparer ladite valeur du rythme cardiaque par rapport à un deuxième seuil prédéterminé ;
- des troisièmes moyens (53) d'analyse des flux sanguins ascendant et descendant mesurés par ledit troisième capteur (33), aptes à comparer ladite valeur du flux sanguin ascendant par rapport à un troisième seuil prédéterminé et du flux sanguin descendant par rapport à un quatrième seuil prédéterminé ;
- des moyens de délivrance (54) d'une information indiquant la détection ou la non détection d'une anomalie en fonction de ladite analyse desdits taux d'oxygène, rythme cardiaque et flux sanguins ascendant et descendant dudit utilisateur ;
- des moyens (55) de stockage desdites données mesurées desdits premier capteur (31), deuxième capteur (32) et troisième capteur (33).

2. Dispositif (1) selon la revendication 1, **caractérisé en ce que** lesdits moyens (55) de stockage contiennent en outre au moins une information relative audit utilisateur appartenant au groupe comprenant : l'âge dudit utilisateur ; une donnée anatomique dudit utilisateur ; une donnée morphologique dudit utilisateur ; un traitement en cours pris par ledit utilisateur ; une pathologie dudit utilisateur ; les antécédents médicaux dudit utilisateur ; le groupe sanguin dudit utilisateur ; les ablations subies par l'utilisateur ; les opérations majeures subies par l'utilisateur ; le groupe sanguin de l'utilisateur ; les résultats de derniers examens (prise de sang, etc.).

3. Dispositif (1) selon la revendication 2, **caractérisé en ce que** :
- le premier seuil prédéterminé est défini en fonction de ladite moins une information relative audit utilisateur, et/ou
- le deuxième seuil prédéterminé est défini en fonction de ladite moins une information relative audit utilisateur, et/ou
- le troisième seuil prédéterminé est défini en fonction de ladite moins une information relative audit utilisateur, et/ou
- le quatrième seuil prédéterminé est défini en fonction de ladite moins une information relative audit utilisateur.

4. Dispositif (1) selon l'une des revendications 1 à 3, **caractérisé en ce qu'**il comprend en outre des moyens d'alerte (6), et **en ce que**, si une information indiquant la détection d'une anomalie est délivrée par lesdits moyens de délivrance (54), lesdits moyens d'alerte (6) génèrent un message d'alerte qui est transmis par le biais de moyens d'émission (7) à au moins un contact prédéterminé, ledit contact prédéterminé étant stocké dans lesdits moyens (55) de stockage.

5. Dispositif (1) selon la revendication 4, **caractérisé en ce que** lesdits moyens d'émission (7) comprennent un port (71) configuré pour recevoir une carte nano SIM.

6. Dispositif (1) selon l'une des revendications 4 ou 5, **caractérisé en ce que** lesdits moyens d'émission (7) comprennent en outre des moyens (72) d'appel d'urgence dudit au moins un contact prédéterminé.

7. Dispositif (1) selon l'une des revendications 1 à 6, **caractérisé en ce qu'**il comprend en outre des moyens (56) d'identification uniques.

8. Dispositif (1) selon l'une des revendications 1 à 7, **caractérisé en ce qu'**il comprend en outre des moyens (57) de détection d'une mise en place dudit dispositif sur ledit poignet dudit utilisateur.

9. Dispositif (1) selon l'une des revendications 1 à 8, **caractérisé en ce qu'**il comprend en outre un capteur appartient au groupe comprenant :
- un capteur de géolocalisation ;
- un capteur de glycémie ;
- un capteur de température.

10. Dispositif (1) selon l'une des revendications 1 à 9, **caractérisé en ce qu'**il comprend une alimentation électrique.

11. Procédé de surveillance médicale individuelle, ledit procédé étant apte à délivrer une information indiquant la détection ou la non détection d'une anomalie, **caractérisé en ce qu'**il met en œuvre un dispositif (1) de surveillance médicale individuelle, de type montre connectée apte à venir au contact d'un poignet d'un utilisateur selon l'une des revendications 1 à 10, et **en ce que** ledit procédé comprend les étapes suivantes, mises en œuvre par ledit dispositif (1) de surveillance médicale individuelle, lorsque ledit module (3) de mesure est positionné en vis-à-vis d'une artère radiale dudit poignet dudit utilisateur :
- mesure (10) du taux d'oxygène dudit utilisateur au niveau de l'artère radiale dudit utilisateur, à l'aide dudit premier capteur (31) ;
- mesure (11) du rythme cardiaque dudit utilisateur par mesure des vibrations du flux sanguin au niveau de ladite artère radiale, à l'aide dudit deuxième capteur (32) ;
- mesure (12) des flux sanguins ascendant et descendant au niveau de ladite artère radiale, à l'aide dudit troisième capteur (33) ;
- analyse (13) desdits taux d'oxygène, rythme cardiaque et flux sanguins ascendant et descendant dudit utilisateur, de sorte à transmettre des instructions de délivrance d'une information indiquant la détection ou la non détection d'une anomalie ;
- délivrance (14) de ladite information indiquant la détection ou la non détection d'une anomalie ;
- stockage (15) desdits taux d'oxygène, rythme cardiaque et flux sanguins ascendant et descendant mesurés dudit utilisateur,
ladite étape d'analyse (13) comprenant les étapes successives suivantes :
- comparaison (131) du taux d'oxygène mesuré par rapport à un premier seuil prédéterminé ;
- comparaison (132) du rythme cardiaque mesuré par rapport à un deuxième seuil prédéterminé ;
- comparaison (133) de la valeur du flux sanguin ascendant par rapport à un troisième seuil prédéterminé et de la valeur du flux sanguin descendant par rapport à un quatrième seuil prédéterminé ; et
- instruction de délivrance (134) de ladite information indiquant la détection d'une anomalie si le taux d'oxygène, et/ou le rythme cardiaque et/ou les flux sanguins ascendant et/ou descendant dudit utilisateur dépassent respectivement les premier, deuxième, troisième et quatrième seuils.

12. Procédé de surveillance médicale individuelle selon la revendication 11, caractérisé en ce, si une information indiquant la détection d'une anomalie est délivrée par lesdits moyens de délivrance (54), le procédé comprend les étapes successives suivantes :
- génération (16) d'un message d'alerte par lesdits moyens d'alerte (6) ;
- envoi (17) dudit message d'alerte généré audit au moins un contact prédéterminé, par le biais desdits moyens d'émission (7).

13. Procédé de surveillance médicale individuelle selon l'une des revendications 11 ou 12, **caractérisé en ce qu'**il comprend une étape (100) préalable d'authentification.

14. Produit programme d'ordinateur téléchargeable depuis un réseau de communication et/ou stocké sur un support lisible par microprocesseur et/ou exécutable par un microprocesseur, **caractérisé en ce qu'**il comprend des instructions de code de programme pour l'exécution d'un procédé de surveillance médicale individuelle selon l'une des revendications 11 à 13, lorsqu'il est exécuté sur un ordinateur ou un terminal mobile.

15. Médium de stockage lisible par un terminal et non transitoire, stockant un programme d'ordinateur comprenant un jeu d'instructions exécutables par un dispositif selon l'une des revendications 1 à 10 pour mettre en œuvre le procédé selon l'une des revendications 11 à 13.

## Patentansprüche

1. Individuelle medizinische Überwachungsvorrichtung (1) vom Typ verbundene Uhr, die imstande ist, mit einem Handgelenk eines Nutzers in Kontakt zu kommen, umfassend einen Hauptkörper (2), der mit einem Anzeigebildschirm (20) versehen ist, ein Armband (4), das mit dem Hauptkörper (2) verbunden ist, und ein Messmodul (3), das bestimmt ist, sich gegenüber einer radialen Arterie des Handgelenks des Nutzers zu positionieren, wenn die Uhr vom Nutzer getragen wird, wobei das Messmodul (3) umfasst:
- einen ersten Sensor (31), der imstande ist, den Sauerstoffgehalt im Bereich der radialen Arterie des Nutzers kontinuierlich zu messen;
- einen zweiten Sensor (32), der imstande ist, den Herzrhythmus des Nutzers durch Messung der Vibrationen des Blutflusses im Bereich der radialen Arterie zu messen;
- einen dritten Sensor (33), der imstande ist, die aufsteigenden und absteigenden Blutströme im Bereich der radialen Arterie zu messen;
wobei die Vorrichtung (1) ein Verarbeitungsmodul (5) umfasst, das aufweist:
- Erfassungsmittel (50) der gemessenen Daten des ersten Sensors (31), des zweiten Sensors (32) und des dritten Sensors (33);
- ersten Analysemittel (51) des von dem ersten Sensor (31) gemessenen Sauerstoffgehalts, die imstande sind, den Wert des Sauerstoffgehalts mit einem ersten vorher festgelegten Grenzwert zu vergleichen;
- zweite Analysemittel (52) der von dem zweiten Sensor (32) gemessenen Anzahl der Vibrationen des Blutflusses, die imstande sind, den Wert des Herzrhythmus mit einem zweiten vorher festgelegten Grenzwert zu vergleichen;
- dritte Analysemittel (53) des von dem dritten Sensor (33) gemessenen aufsteigenden und absteigenden Blutstroms, die imstande sind, den Wert des aufsteigenden Blutstroms mit einem dritten vorher festgelegten Grenzwert und des absteigenden Blutstroms mit einem vierten vorher festgelegten Grenzwert zu vergleichen;
- Bereitstellungsmittel (54) einer Information, die die Feststellung oder die Nichtfeststellung einer Anomalie in Abhängigkeit von der Analyse des Sauerstoffgehalts, des Herzrhythmus und des aufsteigenden und absteigenden Blutstroms des Nutzers anzeigen;
- Speichermittel (55) der gemessenen Daten des ersten Sensors (31), des zweiten Sensors (32) und des dritten Sensors (33).

2. Vorrichtung (1) nach Anspruch 1, **dadurch gekennzeichnet, dass** die Speichermittel (55) ferner mindestens eine sich auf den Nutzer beziehenden Information enthalten, die zu der Gruppe gehört, umfassend: das Alter des Nutzers; eine anatomische Angabe des Nutzers; eine morphologische Angabe des Nutzers; eine Behandlung, die der Nutzer derzeit durchführt; eine Krankheit des Nutzers; die medikamentöse Vorgeschichte des Nutzers; die Blutgruppe des Nutzers; die Ablationen, denen der Nutzer unterzogen wurde; die wichtigsten Operationen, denen der Nutzer unterzogen wurde; die Blutgruppe des Nutzers; die Ergebnisse der letzten Untersuchungen (Blutabnahme usw.).

3. Vorrichtung (1) nach Anspruch 2, **dadurch gekennzeichnet, dass**:
- der erste vorher festgelegte Grenzwert in Abhängigkeit von der wenigstens einen Information festgelegt ist, die sich auf den Nutzer bezieht, und/oder
- der zweite vorher festgelegte Grenzwert in Abhängigkeit von der wenigstens einen Information festgelegt ist, die sich auf den Nutzer bezieht, und/oder
- der dritte vorher festgelegte Grenzwert in Abhängigkeit von der wenigstens einen Information festgelegt ist, die sich auf den Nutzer bezieht, und/oder
- der vierte vorher festgelegte Grenzwert in Abhängigkeit von der wenigstens einen Information festgelegt ist, die sich auf den Nutzer bezieht.

4. Vorrichtung (1) nach einem der Ansprüche 1 bis 3, **dadurch gekennzeichnet, dass** sie ferner Warnmittel (6) umfasst und dass, wenn eine Information, die die Detektion einer Anomalie anzeigt, von den Bereitstellungsmitteln (54) bereitgestellt wird, die Warnmittel (6) eine Warnmeldung erzeugen, die über Sendemittel (7) an mindestens einen vorher festgelegten Kontakt übermittelt werden, wobei der vorher festgelegte Kontakt in den Speichermitteln (55) gespeichert ist.

5. Vorrichtung (1) nach Anspruch 4, **dadurch gekennzeichnet, dass** die Sendemittel (7) einen Port (71) umfassen, der ausgelegt ist, um eine Nano-SIM-Karte aufzunehmen.

6. Vorrichtung (1) nach einem der Ansprüche 4 oder 5, **dadurch gekennzeichnet, dass** die Sendemittel (7) ferner Notrufmittel (72) des mindestens einen vorher festgelegten Kontakts umfassen.

7. Vorrichtung (1) nach einem der Ansprüche 1 bis 6, **dadurch gekennzeichnet, dass** sie ferner einzige Identifikationsmittel (56) umfasst.

8. Vorrichtung (1) nach einem der Ansprüche 1 bis 7, **dadurch gekennzeichnet, dass** sie ferner Detektionsmittel (57) einer Platzierung der Vorrichtung auf dem Handgelenk des Nutzers umfasst.

9. Vorrichtung (1) nach einem der Ansprüche 1 bis 8, **dadurch gekennzeichnet, dass** sie ferner einen Sensor umfasst, der zu der Gruppe gehört, die umfasst:
- einen Geolokalisierungssensor;
- einen Glykämiesensor;
- einen Temperatursensor.

10. Vorrichtung (1) nach einem der Ansprüche 1 bis 9, **dadurch gekennzeichnet, dass** sie eine Stromversorgung umfasst.

11. Verfahren zur individuellen medizinischen Überwachung, wobei das Verfahren imstande ist, eine Information bereitzustellen, die die Detektion oder die Nichtdetektion einer Anomalie anzeigt, **dadurch gekennzeichnet, dass** es eine individuelle medizinische Überwachungsvorrichtung (1) vom Typ verbundene Uhr verwendet, die imstande ist, mit einem Handgelenk eines Nutzers nach einem der Ansprüche 1 bis 10 in Kontakt zu kommen und dass das Verfahren die folgenden Schritte umfasst, die von der individuellen medizinischen Überwachungsvorrichtung (1) durchgeführt werden, wenn das Messmodul (3) gegenüber einer radialen Arterie des Handgelenks des Nutzers positioniert ist:
- Messen (10) des Sauerstoffgehalts des Nutzers im Bereich der radialen Arterie des Nutzers mit Hilfe des ersten Sensors (31);
- Messen (11) des Herzrhythmus des Nutzers durch Messen der Vibrationen des Blutflusses im Bereich der radialen Arterie mit Hilfe des zweiten Sensors (32);
- Messen (12) des aufsteigenden und absteigenden Blutstroms im Bereich der radialen Arterie mit Hilfe des dritten Sensors (33);
- Analyse (13) von Sauerstoffgehalt, Herzrhythmus und des aufsteigenden und absteigenden Blutstroms des Nutzers derart, dass Befehle für die Bereitstellung einer Information übertragen werden, die die Detektion oder die Nichtdetektion einer Anomalie anzeigt;
- Bereitstellen (14) der Information, die die Detektion oder die Nichtdetektion einer Anomalie anzeigt;
- Speichern (15) des gemessenen Sauerstoffgehalts, Herzrhythmus und aufsteigenden und absteigenden Blutstroms des Nutzers,
wobei der Analyseschritt (13) die folgenden aufeinanderfolgenden Schritte umfasst:
- Vergleichen (131) des gemessenen Sauerstoffgehalts mit einem ersten vorher festgelegten Grenzwert;
- Vergleichen (132) des gemessenen Herzrhythmus mit einem zweiten vorher festgelegten Grenzwert;
- Vergleichen (133) des Werts des aufsteigenden Blutstroms mit einem dritten vorher festgelegten Grenzwert und des Werts des absteigenden Blutstroms mit einem vierten vorher festgelegten Grenzwert; und
- Befehl des Bereitstellens (134) der Information, die die Detektion einer Anomalie anzeigt, wenn der Sauerstoffgehalt und/oder der Herzrhythmus und/oder der aufsteigende und/oder absteigende Blutstrom des Nutzers jeweils den ersten, zweiten, dritten und vierten Grenzwert überschreiten.

12. Verfahren zur individuellen medizinischen Überwachung nach Anspruch 11, **dadurch gekennzeichnet, dass**, wenn eine Information, die die Detektion einer Anomalie anzeigt, von den Bereitstellungsmitteln (54) bereitgestellt wird, das Verfahren die folgenden aufeinanderfolgenden Schritte umfasst:
- Erzeugen (16) einer Warnmeldung durch die Warnmittel (6);
- Versenden (17) der erzeugten Warnmeldung an den mindestens einen vorher festgelegten Kontakt über die Sendemittel (7).

13. Verfahren zur individuellen medizinischen Überwachung nach einem der Ansprüche 11 oder 12, **dadurch gekennzeichnet, dass** es einen vorherigen Authentifizierungsschritt (100) umfasst.

14. Rechnerprogrammprodukt, das von einem Kommunikationsnetz herunterladbar und/oder auf einem mikroprozessorlesbaren Medium gespeichert und/oder von einem Mikroprozessor ausführbar ist, **dadurch gekennzeichnet, dass** es Programmcodebefehle für die Ausführung eines individuellen medizinischen Überwachungsverfahrens nach einem der Ansprüche 11 bis 13 umfasst, wenn es auf einem Rechner oder einem mobilen Endgerät ausgeführt wird.

15. Speichermedium, das von einem Endgerät lesbar und nicht transitorisch ist, das ein Rechnerprogramm speichert, das einen Satz an Befehlen umfasst, die von einer Vorrichtung nach einem der Ansprüche 1 bis 10 ausführbar sind, um das Verfahren nach einem der Ansprüche 11 bis 13 durchzuführen.

## Claims

1. An individual medical monitoring device (1), of the smartwatch type, able to come into contact with a wrist of a user, comprising a main body (2) provided with a display screen (20), a bracelet (4) connected to the main body (2), and a measuring module (3) intended to be positioned opposite a radial artery of the wrist of the user, when the watch is worn by the user, said measuring module (3) comprising:
- a first sensor (31) able to continuously measure the oxygen level at the radial artery of said user;
- a second sensor (32) able to measure the heart rate of said user by measuring the vibrations of the blood flow at said radial artery;
- a third sensor (33) able to measure the ascending and descending blood flows at said radial artery;
said device (1) comprising a processing module (5) including:
- means (50) for acquiring the measured data of said first sensor (31), second sensor (32) and third sensor (33) ;
- first means (51) for analysing the oxygen level measured by said first sensor (31), able to compare said value of the oxygen level with respect to a first predetermined threshold;
- second means (52) for analysing the number of vibrations of the blood flow measured by said second sensor (32), able to compare said value of the heart rate with respect to a second predetermined threshold;
- third means (53) for analysing the ascending and descending blood flows measured by said third sensor (33), able to compare said value of the ascending blood flow with respect to a third predetermined threshold and of the descending blood flow with respect to a fourth predetermined threshold;
- means for outputting (54) a piece of information indicating the detection or the non-detection of an anomaly as a function of said analysis of said oxygen levels, heart rate and ascending and descending blood flows of said user;
- means (55) for storing said measured data of said first sensor (31), second sensor (32) and third sensor (33).

2. The device (1) according to claim 1, **characterised in that** said storage means (55) further contain at least one piece of information relating to said user belonging to the group comprising: the age of said user; an anatomical piece of data of said user; a morphological piece of data of said user; a current treatment taken by said user; a pathology of said user; the medical history of said user; the blood group of said user; the ablations the user has undergone; the major operations the user has undergone; the blood group of the user; the results of the last examinations (blood sample, etc.).

3. The device (1) according to claim 2, **characterised in that**:
- the first predetermined threshold is defined as a function of said at least one piece of information relating to said user, and/or
- the second predetermined threshold is defined as a function of said at least one piece of information relating to said user, and/or
- the third predetermined threshold is defined as a function of said at least one piece of information relating to said user, and/or
- the fourth predetermined threshold is defined as a function of said at least one piece of information relating to said user.

4. The device (1) according to one of claims 1 to 3, **characterised in that** it further comprises alert means (6), and **in that**, if a piece of information indicating the detection of an anomaly is output by said output means (54), said alert means (6) generate an alert message which is transmitted via emission means (7) to at least one predetermined contact, said predetermined contact being stored in said storage means (55).

5. The device (1) according to claim 4, **characterised in that** said emission means (7) comprise a port (71) configured to receive a Nano SIM card.

6. The device (1) according to one of claims 4 or 5, **characterised in that** said emission means (7) further comprise means (72) for emergency calling said at least one predetermined contact.

7. The device (1) according to one of claims 1 to 6, **characterised in that** it further comprises unique identification means (56).

8. The device (1) according to one of claims 1 to 7, **characterised in that** it further comprises means (57) for detecting a placement of said device on said wrist of said user.

9. The device (1) according to one of claims 1 to 8, **characterised in that** it further comprises a sensor belonging to the group comprising:
- a geolocation sensor;
- a blood glucose sensor;
- a temperature sensor.

10. The device (1) according to one of claims 1 to 9, **characterised in that** it comprises an electric power supply.

11. An individual medical monitoring method, said method being able to output a piece of information indicating the detection or the non-detection of an anomaly, **characterised in that** it implements an individual medical monitoring device (1), of the smartwatch type able to come into contact with a wrist of a user according to one of claims 1 to 10, and **in that** said method comprises the following steps, implemented by said individual medical monitoring device (1), when said measuring module (3) is positioned opposite a radial artery of said wrist of said user:
- measurement (10) of the oxygen level of said user at the radial artery of said user, using said first sensor (31);
- measurement (11) of the heart rate of said user by measuring the vibrations of the blood flow at said radial artery, using said second sensor (32);
- measurement (12) of the ascending and descending blood flows at said radial artery, using said third sensor (33);
- analysis (13) of said oxygen levels, heart rate and ascending and descending blood flows of said user, so as to transmit instructions for outputting a piece of information indicating the detection or the non-detection of an anomaly;
- output (14) of said information indicating the detection or the non-detection of an anomaly;
- storage (15) of said measured oxygen levels, heart rate and ascending and descending blood flows of said user, said analysis step (13) comprising the following successive steps:
- comparison (131) of the measured oxygen level with respect to a first predetermined threshold;
- comparison (132) of the measured heart rate with respect to a second predetermined threshold;
- comparison (133) of the value of the ascending blood flow with respect to a third predetermined threshold and the value of the descending blood flow with respect to a fourth predetermined threshold; and
- instruction to output (134) said information indicating the detection of an anomaly if the oxygen level, and/or the heart rate and/or the ascending and/or descending blood flows of said user respectively exceed the first, second, third and fourth thresholds.

12. The individual medical monitoring method according to claim 11, **characterised in that**, if a piece of information indicating the detection of an anomaly is output by said output means (54), the method comprises the following successive steps:
- generation (16) of an alert message by said alert means (6) ;
- sending (17) of said generated alert message to said at least one predetermined contact, via said emission means (7) .

13. The individual medical monitoring method according to one of claims 11 or 12, **characterised in that** it comprises a prior authentication step (100).

14. A computer program product downloadable from a communication network and/or stored on a computer-readable medium and/or executable by a microprocessor, **characterised in that** it comprises program code instructions for the execution of an individual medical monitoring method according to one of claims 11 to 13, when it is executed on a computer or a mobile terminal.

15. A non-transitory terminal-readable storage medium, storing a computer program comprising a set of instructions executable by a device according to one of claims 1 to 10 to implement the method according to one of claims 11 to 13.
